# EUROPEAN PATENT APPLICATION

(11) **EP 4 813 411 A1**
(43) Date of publication of application: **30.09.2026**
(21) Application number: 24894442.3
(22) Date of filing: 31.10.2024
(51) Int. Cl.: A61L 27/52, A61L 27/36

(54) **COMPOSITION FOR DELAYING IN VIVO DEGRADATION RATE AND REINFORCING RHEOLOGICAL PROPERTIES OF HYALURONIC ACID HYDROGEL FOR BIOTRANSPLANTATION**

(30) Priority: 22.11.2023 KR 20230163597
(71) Applicant: Recm Bio Co.,Ltd, Busan 48059 (KR)
(72) Inventor: KIM, Changsoo, Seoul 06526 (KR); OH, Joonsuk, Seoul 06526 (KR); KIM, Sujin, Seoul 06526 (KR); KIM, Do Hyun, Seoul 06526 (KR); KIM, Jina, Seoul 06526 (KR)
(74) Representative: Vossius & Partner Patentanwälte Rechtsanwälte mbB
(86) International application number: PCT/KR2024/016948
(87) International publication number: WO 2025/110548

(57) **Abstract**

The present disclosure relates to a composition for delaying the *in vivo* degradation rate and reinforcing the rheological properties of a hyaluronic acid hydrogel for bio-implantation, and particularly, to a composition including particulated human acellular dermal matrix that may be mixed on-site with a hyaluronic acid hydrogel used in bio-implantation for tissue repair and having a storage modulus (G') of 300 Pa or less, thereby delaying the *in vivo* degradation rate of hyaluronic acid, relatively increasing the storage modulus (G') without adding a cross-linking agent, and providing an injection force that ensures high user convenience.

## Description

### Technical Field

The present disclosure relates to a composition for delaying the *in vivo* degradation rate and reinforcing the rheological properties of a hyaluronic acid hydrogel for bio-implantation, and particularly, to a composition including particulated human acellular dermal matrix that may be mixed on-site with a hyaluronic acid hydrogel used in bio-implantation for tissue repair and having a storage modulus (G') of 300 Pa or less.

### Background Art

Hyaluronic acid (HA) is a biosynthesized natural substance present in various tissues within the body, such as the skin. Due to the excellent biocompatibility, moisturizing properties, and physical properties thereof, HA is widely used in the form of hydrogels in the biomedical application fields, such as medical devices for tissue repair (e.g., facial contouring fillers) and intra-articular injections.

However, HA-based hydrogel products have limitations in maintaining functional persistence as biomaterials due to rapid *in vivo* degradation. To overcome these limitations, HA-based hydrogel products are typically commercialized with enhanced *in vivo* stability through chemical cross-linking using cross-linking agents such as 1,4-butanediol diglycidyl ether (BDDE), polyethylene glycol diglycidyl ether (PEGDE), and divinyl sulfone (DVS), and the rheological properties of cross-linked HA hydrogels, as biomaterials for tissue repair, are significantly influenced by cross-linking density thereof.

The required rheological properties of HA hydrogels for skin injection vary depending on the target site. For example, a composition having a relatively high storage modulus (G') is required for regions such as the forehead and the nasal bridge. Current commercialized products incorporate a high concentration of HA in finished products or use more cross-linking agents during preparation. Increasing the concentration of HA may lead to an increase in injection force during procedures or difficulties in content control after a washing process. In addition, increasing the amount of a cross-linking agent during preparation may cause issues related to the safety of biomaterials.

Therefore, there is a need for an HA-based hydrogel composition and a method of preparing the same, which can delay the *in vivo* degradation and maintain a relatively high storage modulus (G') of HA without the addition of more cross-linking agents during preparation.

Meanwhile, skin grafts are classified into human-derived allogeneic dermal matrices, animal-derived xenogeneic dermal matrices, and synthetic materials. Although human acellular dermal matrix-based skin grafts are relatively expensive, these are evaluated as optimal substitutes due to the decellularized state, which prevents immunological rejection or inflammatory responses after transplantation. Extracellular matrix (ECM) components, such as collagen, elastin, and fibronectin contained in the dermis, are essential cellular elements that facilitate cell adhesion and proliferation, thereby providing skin regeneration effects.

"Sheet-type human acellular dermal matrix" refers to dermis obtained by removing cells capable of eliciting immunological rejection from isolated allogeneic dermal matrix through chemical treatment, and mainly includes collagen and elastin. Sheet-type acellular dermal matrix tissue is manufactured in various areas according to a target disease site and is used in a grafting or implantation manner.

Particulated human acellular dermal matrix (phADM) refers to a particulate structure produced by pulverizing sheet-type human acellular dermal matrix. Conventionally, lyophilized particulated human acellular dermal matrix was hydrated with saline or distilled water for use as a skin graft, and had the advantage of being injectable via a syringe without surgery due to the maintenance of its flowable state. For this reason, particulated human acellular dermal matrix tissue is used not only for the treatment of skin tissue defects caused by accidents, but also for tissue repair or tissue reconstruction, such as the treatment of diabetic ulcers and chronic diseases. Most current commercially available particulated human dermal matrices have an average particle size of 500 µm or more, which requires large-diameter needles when injected for plastic surgery and aesthetic purposes, and have limitations in achieving homogeneous dispersion when mixed with commercially available biomaterials for tissue repair, such as HA fillers.

Korean Registered Patent Publication No. 10-1523878 discloses a method of preparing a composition for bio-implantation in which particulated human acellular dermal matrix and hyaluronic acid are cross-linked. However, the particulated human acellular dermal matrix used in the present disclosure has a particle size of 300-800 µm, which makes it difficult to use for injection for plastic surgery and aesthetic purposes. In addition, the present disclosure is in a form where hyaluronic acid is cross-linked to the surface of particulated human acellular dermal matrix, which is significantly different from a form in which acellular dermal matrix is physically mixed with a commercially available hyaluronic acid hydrogel.

To date, there have been no cases of facilitating injection as a biomaterial for tissue repair by reducing the particle size of particulated human acellular dermal matrix and mixing the matrix with hyaluronic acid hydrogel. Furthermore, there are no known studies that have enhanced the rheological properties of a composition by addressing the shortcomings of commercially available hyaluronic acid hydrogel products without cross-linking or other reactions.

While some literature discloses finished compositions including human acellular dermal matrix and hyaluronic acid hydrogel, there have been no studies on a method of mixing acellular dermal matrix that is prepared directly at a medical site for the purpose of delaying the *in vivo* degradation and enhancing the rheological properties of commercially available hyaluronic acid hydrogel products.

Therefore, the inventors of the present disclosure have established a method of mixing particulated human acellular dermal matrix with commercially available hyaluronic acid hydrogel products at a medical site, and identified conditions for delaying the *in vivo* degradation and enhancing the rheological properties of HA without the addition of cross-linking agents, thereby completing the present disclosure. Specifically, conditions for delaying the *in vivo* degradation and enhancing the rheological properties of HA were identified by specifying the particle size of particulated human acellular dermal matrix, the relative ratio of the particle size of HA to the particle size of particulated human acellular dermal matrix, the mixing concentration of the particulated human acellular dermal matrix, and the storage modulus (G') of a commercially available hyaluronic acid hydrogel.

### Disclosure of Invention

### Technical Problem

A first objective of the present disclosure is to provide a composition for delaying the *in vivo* degradation rate and reinforcing the rheological properties of a hyaluronic acid hydrogel used in bio-implantation for tissue repair.

A second objective of the present disclosure is to provide a bio-implantable composition for tissue repair in the form of an on-site formulation.

A third objective of the present disclosure is to provide a bio-implantable composition for tissue repair in a finished form.

### Solution to Problem

In the present disclosure, a method of mixing particulated human acellular dermal matrix with a commercially available hyaluronic acid hydrogel product at a medical site was established, and conditions were identified for delaying the *in vivo* degradation and enhancing the rheological properties of hyaluronic acid without the addition of a cross-linking agent. Specifically, conditions for delaying the *in vivo* degradation and enhancing the rheological properties of HA were identified by specifying the particle size of particulated human acellular dermal matrix, the relative ratio of the particle size of HA to the particle size of the particulated human acellular dermal matrix, the mixing concentration of the particulated human acellular dermal matrix, and the storage modulus (G') of a commercially available hyaluronic acid hydrogel.

The present disclosure may particularly increase procedure satisfaction when applied to a treatment site (e.g., the forehead, the nasal bridge, or the like) requiring a relatively high storage modulus (G'), and may also provide an effect of extending the *in vivo* retention period of a biomaterial graft for tissue repair.

### Composition for delaying in vivo degradation rate and reinforcing rheological properties of hyaluronic acid hydrogel

The present disclosure provides a composition for delaying the *in vivo* degradation rate and reinforcing the rheological properties of a hyaluronic acid hydrogel
used in bio-implantation for tissue repair and having a storage modulus (G') of 300 Pa or less, the composition including particulated human acellular dermal matrix and a medical diluting solvent.

In the reinforcing composition according to the present disclosure, the hyaluronic acid hydrogel is a hyaluronic acid hydrogel that requires delay of the *in vivo* degradation rate or reinforcement of the rheological properties, and may be a commercially available product already in use at a medical site or a product scheduled for commercialization.

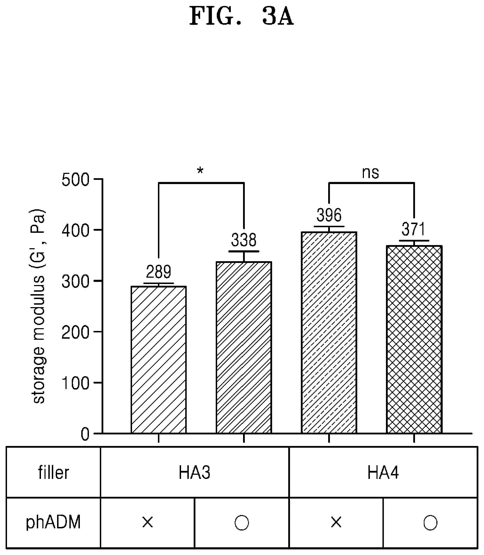

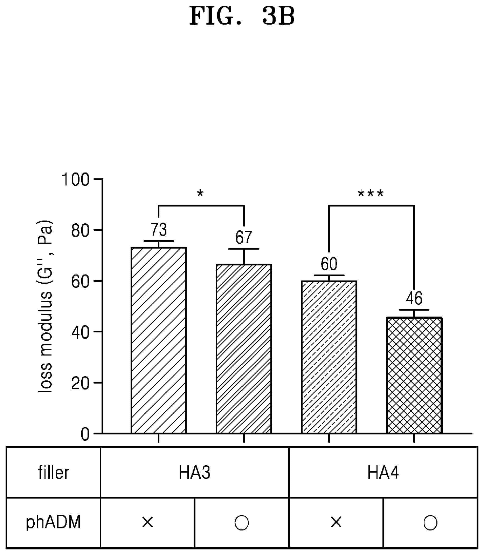

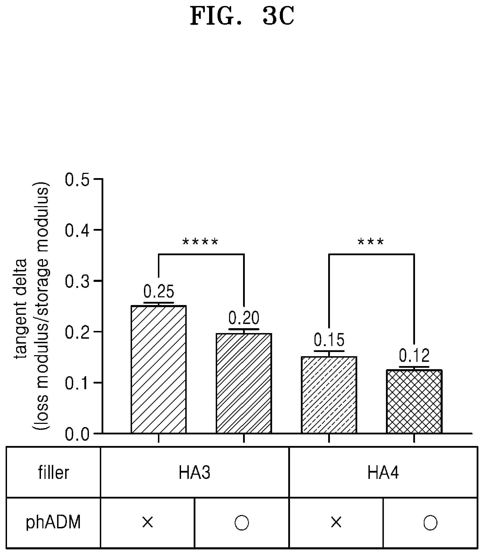

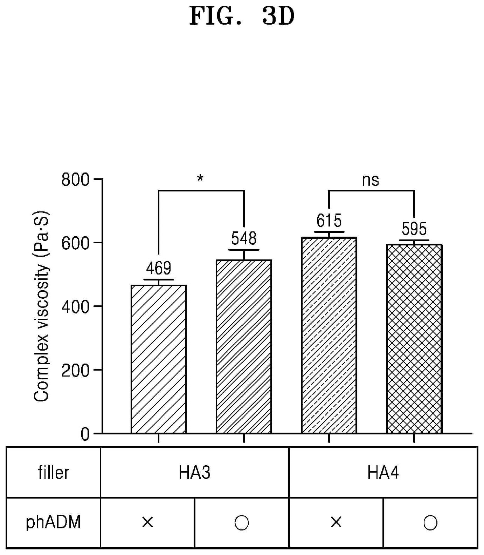

In the present disclosure, the rheological properties (storage modulus, G') may be enhanced by optionally mixing a hyaluronic acid hydrogel having a storage modulus (G') of 300 Pa or less with particulated human acellular dermal matrix. In case that a hyaluronic acid hydrogel having a storage modulus (G') exceeding 300 Pa is used, the storage modulus (G') may instead be reduced (see FIG. 3 and Table 4).

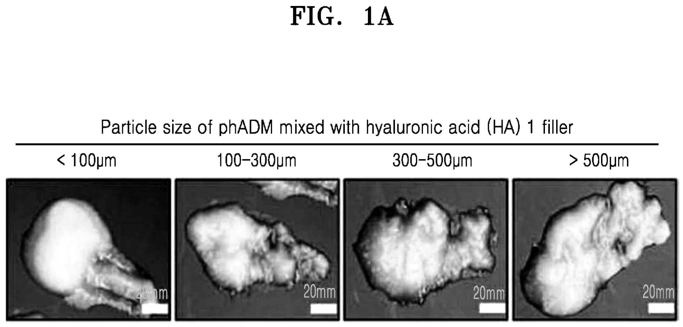

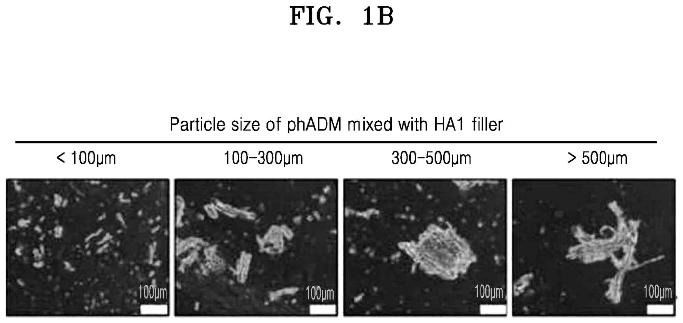

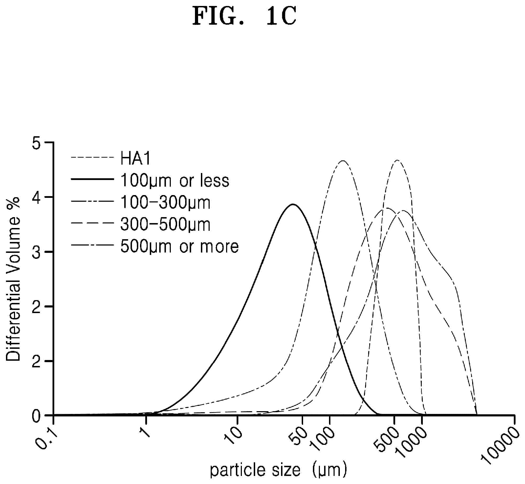

In the reinforcing composition according to the present disclosure, the particulated human acellular dermal matrix may have an average particle size of 300 µm or less, specifically 200 µm or less, and more specifically 100 µm or less. In case that particulated human acellular dermal matrix having particle size exceeding 300 µm is used, the particulated human acellular dermal matrix may not be homogeneously mixed with hyaluronic acid hydrogel (see Table 1 and FIG. 1).

In other embodiments, the particle size of the particulated human acellular dermal matrix may be 1/3 or less, preferably 1/5 or less, of an average particle size of hyaluronic acid. In case that the particle size of the particulated human acellular dermal matrix exceeds 1/3 of the average particle size of hyaluronic acid, the particulated human acellular dermal matrix may not be homogeneously mixed with the hyaluronic acid hydrogel (see Table 1 and FIG. 1).

In the reinforcing composition according to the present disclosure, the medical diluting solvent may be one of normal saline, distilled water, phosphate-buffered saline (PBS), and the like, or a mixture of two or more thereof, and preferably, normal saline, which is most widely used in medical sites, may be used.

In the reinforcing composition according to the present disclosure, a mixed composition of the reinforcing composition and hyaluronic acid hydrogel may include the particulated human acellular dermal matrix in an amount of 10 wt% or less, specifically 1-10 wt%, and more specifically 1-5 wt%. In case that the amount of the particulated human acellular dermal matrix exceeds 10 wt%, a rapid increase in injection force may occur, making the procedure difficult (see Table 2). In case that the amount of the particulated human acellular dermal matrix is less than 1 wt%, the effects of delaying the *in vivo* degradation and enhancing the rheological properties of hyaluronic acid may be insignificant. In other embodiments, by mixing the reinforcing composition according to the present disclosure with hyaluronic acid hydrogel, the *in vivo* degradation rate of hyaluronic acid by hyaluronidase may be significantly reduced. In case that the particulated human acellular dermal matrix is included in an amount exceeding 10 wt% in the mixed composition, the effect of reducing the degradation rate of hyaluronic acid may gradually decrease (see Table 3).

For reference, when hyaluronic acid hydrogel is used as a biomaterial for tissue repair in aesthetic plastic surgery, it is common to set a standard of 40 N or less when connected to a needle, considering factors such as injection convenience (Guidelines for Approval and Review of Biomaterials for Plastic Tissue Repair, Ministry of Food and Drug Safety, 2020).

The rheological properties mean an increase in storage modulus (G'). The rheological properties also mean a decrease in tan delta (tan δ) and an increase in complex viscosity.

### Bio-implantable composition for tissue repair in form of on-site formulation

The present disclosure provides a bio-implantable composition for tissue repair,
including particulated human acellular dermal matrix,
in which the bio-implantable composition is administered in the form of a mixed composition prepared by mixing a solution, in which the particulated human acellular dermal matrix is mixed in a medical diluting solvent, with hyaluronic acid hydrogel on-site, and
the hyaluronic acid hydrogel has a storage modulus (G') of 300 Pa or less.

In the bio-implantable composition for tissue repair according to the present disclosure, the medical diluting solvent may be one of normal saline, distilled water, phosphate-buffered saline (PBS), and the like, or a mixture of two or more thereof, and preferably, normal saline, which is most widely used in medical sites, may be used.

Specifically,
The particulated human acellular dermal matrix may have an average particle size of 300 µm or less,
the mixed composition may include 0.5-10 wt% of the particulated human acellular dermal matrix,
the mixed composition may include 0.5-2 wt%of the hyaluronic acid hydrogel, and
the average particle size of the particulated human acellular dermal matrix may be 1/3 or less of an average particle size of hyaluronic acid.

Specifically,
The particulated human acellular dermal matrix may have an average particle size of 200 µm or less,
the mixed composition may include 1-10 wt% of the particulated human acellular dermal matrix,
the mixed composition may include 0.7-1.5 wt% of the hyaluronic acid hydrogel, and
the average particle size of the particulated human acellular dermal matrix may be 1/3 or less of an average particle size of hyaluronic acid.

Specifically,
The particulated human acellular dermal matrix may have an average particle size of 100 µm or less,
the mixed composition may include 1-5 wt% of the particulated human acellular dermal matrix,
the mixed composition may include 0.8-1.4 wt% of the hyaluronic acid hydrogel, and
the average particle size of the particulated human acellular dermal matrix may be 1/3 or less of an average particle size of hyaluronic acid.

Specifically,
the particulated human acellular dermal matrix may have an average particle size of 100 µm or less,
the mixed composition may include 1-5 wt% of the particulated human acellular dermal matrix,
the mixed composition may include 0.9-1.3 wt% of the hyaluronic acid hydrogel, and
the average particle size of the particulated human acellular dermal matrix may be 1/3 or less of an average particle size of hyaluronic acid.

Specifically,
the particulated human acellular dermal matrix may have an average particle size of 100 µm or less,
the mixed composition may include 1-5 wt% of the particulated human acellular dermal matrix,
the mixed composition may include 1-1.2 wt% of the hyaluronic acid hydrogel, and
the average particle size of the particulated human acellular dermal matrix may be 1/3 or less of an average particle size of hyaluronic acid.

### Bio-implantable composition for tissue repair in finished form

It is obvious that the conditions identified in the present disclosure for delaying the *in vivo* degradation and enhancing the rheological properties of hyaluronic acid are also applicable to a finished composition including particulated human acellular dermal matrix and hyaluronic acid hydrogel.

Therefore, the present disclosure provides a bio-implantable composition for tissue repair, including: a solution in which particulated human acellular dermal matrix and a medical diluting solvent are mixed; and
a hyaluronic acid hydrogel having a storage modulus (G') of 300 Pa or less.

In the composition for tissue repair according to the present disclosure,
the particulated human acellular dermal matrix may have an average particle size of 300 µm or less,
the composition may include 0.5-15 wt% of the particulated human acellular dermal matrix,
the composition may include 0.5-2 wt% of the hyaluronic acid hydrogel, and
the average particle size of the particulated human acellular dermal matrix may be 1/3 or less of an average particle size of hyaluronic acid.

### Advantageous Effects of Invention

According to the present disclosure, a composition for delaying the *in vivo* degradation rate and reinforcing the rheological properties of a hyaluronic acid hydrogel, which is used in bio-implantation for tissue repair and has a storage modulus (G') of 300 Pa or less, includes particulated human acellular dermal matrix that is mixed with the hyaluronic acid hydrogel, thereby delaying the *in vivo* degradation rate of hyaluronic acid, relatively increasing the storage modulus (G') without adding a cross-linking agent, and providing an injection force that ensures high user convenience.

### Brief Description of Drawings

FIG. 1A illustrates observation images showing the appearance of samples prepared such that the average particle sizes of particulated human acellular dermal matrix in mixed compositions of the particulated human acellular dermal matrix and hyaluronic acid were 100 µm or less, 100 to 300 µm, 300 to 500 µm, and 500 µm or more.
FIG. 1B illustrates optical microscopic observation results showing the shapes of phADM particles in samples prepared such that the average particle sizes of particulated human acellular dermal matrix in mixed compositions of the particulated human acellular dermal matrix and hyaluronic acid were 100 µm or less, 100 to 300 µm, 300 to 500 µm, and 500 µm or more.
FIG. 1C is a graph comparing the particle size distributions of a hyaluronic acid sample (Sample name: HA1, manufacturer: Hugel, Korea) and four types of human acellular dermal matrix powders with different average particle sizes, within samples prepared such that the average particle sizes of particulated human acellular dermal matrix in mixed compositions of the particulated human acellular dermal matrix and hyaluronic acid were 100 µm or less, 100 to 300 µm, 300 to 500 µm, and 500 µm or more.

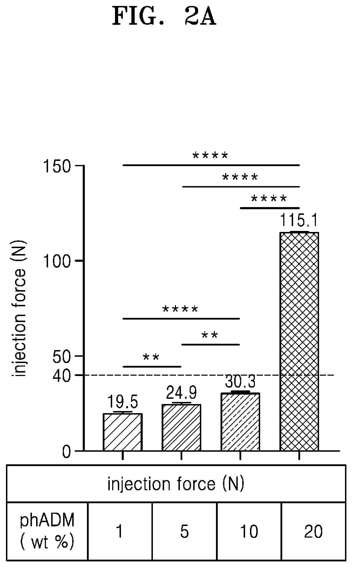

FIG. 2A illustrates the results of measuring the injection forces of syringes, each equipped with a commonly used 23G (thin-wall) needle and filled with a mixed composition sample of particulated human acellular dermal matrix (1, 5, 10, or 20 wt%) and hyaluronic acid (Sample name: HA2, manufacturer: Hugel, Korea) (1 wt%).

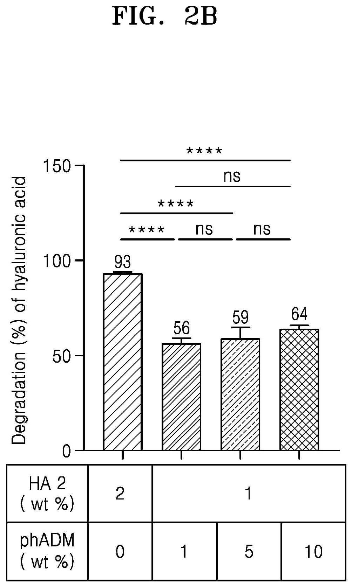

FIG. 2B illustrates the results of measuring the degradation rate of hyaluronic acid within a mixed composition sample of particulated human acellular dermal matrix (0, 1, 5, or 10 wt%) and hyaluronic acid (Sample name: HA2, manufacturer: Hugel, Korea) (1 wt%), 24 hours after treatment with hyaluronidase.
   Data in FIG. 2 = mean ± standard deviation; and *p < 0.05, **p < 0.01, ***p < 0.001, and ****p < 0.0001.
FIG. 3A illustrates the results of measuring the storage modulus (G') before and after mixing phADM having an average particle size of 100 µm or less with each of the hyaluronic acids, HA3 or HA4 (sample name: HA3, manufacturer: Medytox Inc., Korea; and sample name: HA4, manufacturer: Hugel, Korea).
FIG. 3B illustrates the results of measuring the loss modulus (G") before and after mixing phADM having an average particle size of 100 µm or less with each of the hyaluronic acids, HA3 or HA4 (sample name: HA3, manufacturer: Medytox Inc., Korea; and sample name: HA4, manufacturer: Hugel, Korea).
FIG. 3C illustrates the results of measuring the tan delta before and after mixing phADM having an average particle size of 100 µm or less with each of the hyaluronic acids, HA3 or HA4 (sample name: HA3, manufacturer: Medytox Inc., Korea; and sample name: HA4, manufacturer: Hugel, Korea).
FIG. 3D illustrates the results of measuring the complex viscosity before and after mixing phADM having an average particle size of 100 µm or less with each of the hyaluronic acids, HA3 or HA4 (sample name: HA3, manufacturer: Medytox Inc., Korea; and sample name: HA4, manufacturer: Hugel, Korea).
   Data in FIG. 3 = mean ± standard deviation; and *p < 0.05, **p < 0.01, ***p < 0.001, and ****p < 0.0001.

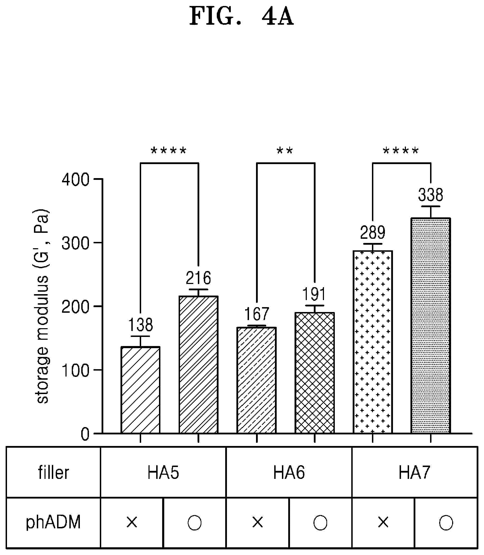

FIG. 4A illustrates the results of measuring the storage modulus (G') before and after mixing phADM having an average particle size of 100 µm or less with each of the hyaluronic acids, HA5 to HA7 (sample name: HA5, manufacturer: Medytox Inc., Korea; sample name: HA6, manufacturer: Jetema Co., Ltd., Korea; and sample name: HA7, manufacturer: Medytox Inc., Korea).

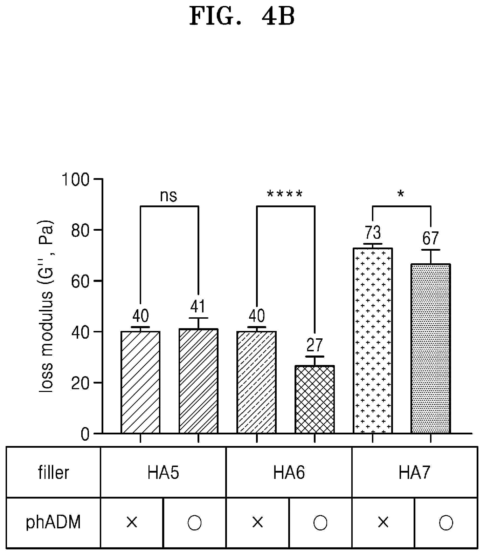

FIG. 4B illustrates the results of measuring the loss modulus (G") before and after mixing phADM having an average particle size of 100 µm or less with each of the hyaluronic acids, HA5 to HA7 (sample name: HA5, manufacturer: Medytox Inc., Korea; sample name: HA6, manufacturer: Jetema Co., Ltd., Korea; and sample name: HA7, manufacturer: Medytox Inc., Korea).

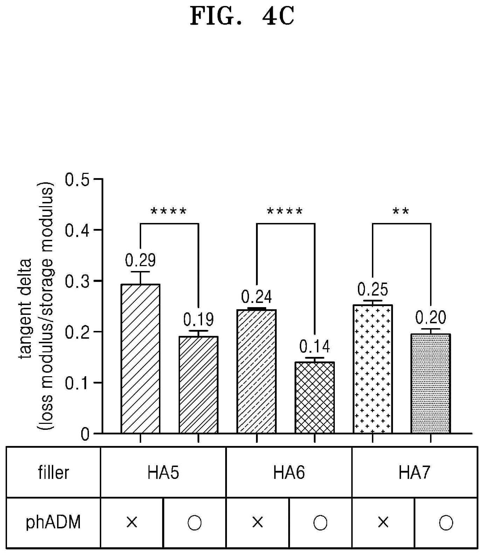

FIG. 4C illustrates the results of measuring the tan delta before and after mixing phADM having an average particle size of 100 µm or less with each of the hyaluronic acids, HA5 to HA7 (sample name: HA5, manufacturer: Medytox Inc., Korea; sample name: HA6, manufacturer: Jetema Co., Ltd., Korea; and sample name: HA7, manufacturer: Medytox Inc., Korea).

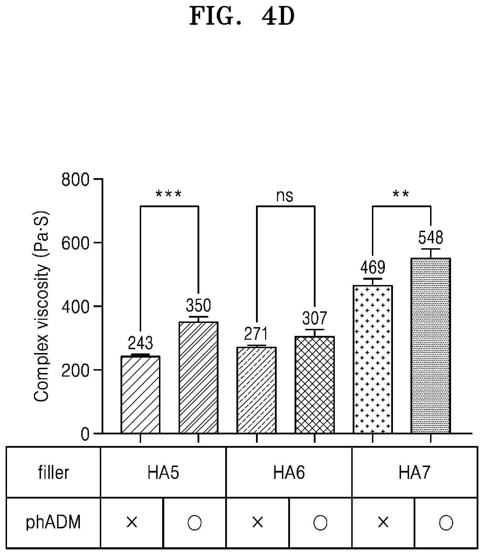

FIG. 4D illustrates the results of measuring the complex viscosity before and after mixing phADM having an average particle size of 100 µm or less with each of the hyaluronic acids, of HA5 to HA7 (sample name: HA5, manufacturer: Medytox Inc., Korea; sample name: HA6, manufacturer: Jetema Co., Ltd., Korea; and sample name: HA7, manufacturer: Medytox Inc., Korea).
   Data in FIG. 4 = mean ± standard deviation; and *p < 0.05, **p < 0.01, ***p < 0.001, and ****p < 0.0001.

### Mode for the Invention

Hereinafter, the present disclosure will be described in detail.

The term "sheet-type human acellular dermal matrix" as used herein refers to a dermal matrix that is obtained by removing cells capable of inducing immunological rejection from isolated allogeneic dermal matrix through chemical treatment, and that mainly includes collagen and elastin. Sheet-type human acellular dermal matrix tissue is produced in various areas according to a target disease site and is used in a grafting or implantation manner.

The term "particulated human acellular dermal matrix (phADM)" as used herein refers to a particulate structure produced by pulverizing sheet-type human acellular dermal matrix. Conventionally, lyophilized particulated human acellular dermal matrix was hydrated with saline or distilled water for use as a skin graft, and had the advantage of being injectable via a syringe without surgery due to the maintenance of its flowable state. For this reason, particulated human acellular dermal matrix tissue is used not only for the treatment of skin tissue defects caused by accidents, but also for tissue repair or tissue reconstruction, such as the treatment of diabetic ulcers and chronic diseases. Most current commercially available particulated human acellular dermal matrices have an average particle size of 500 µm or more, which requires large-diameter needles when injected for aesthetic or plastic surgery purposes, and have limitations in achieving homogeneous dispersion when mixed with commercially available biomaterials for tissue repair, such as existing HA fillers.

The term "hyaluronic acid (HA)" as used herein refers to a biosynthetic natural substance abundantly present in the skin, synovial fluid, cartilage, and the like of animals and the like, and is a hydrophilic mucopolysaccharide due to its high content of hydroxyl groups. Hyaluronic acid binds with water to form a gel, contributing to the lubrication of joints and the flexibility of the skin. Due to its high viscosity, hyaluronic acid also plays a critical role in preventing bacterial invasion and the penetration of toxins into the skin. A deficiency of hyaluronic acid in the skin leads to skin dryness, loss of elasticity, and the formation of wrinkles. For this reason, hyaluronic acid has been extensively used as a biomaterial for tissue repair to prevent wrinkles or to fill depressed areas of the skin. Commercially available hyaluronic acid hydrogel products exhibit differences in physicochemical and biological properties depending on the cross-linking method using BDDE or the like, and the degree of cross-linking.

The term "injection force" as used herein refers to the force exerted when applying pressure through a syringe, and is expressed in unit of [N]. When hyaluronic acid hydrogel is used as a biomaterial for tissue repair in aesthetic plastic surgery, it is common to set a standard of 40 N or less when connected to a needle used, considering factors such as injection convenience (Guidelines for Approval and Review of Biomaterials for Plastic Tissue Repair, Ministry of Food and Drug Safety, 2020).

The term "elastic modulus or storage modulus (G')" as used herein refers to a value representing the elastic response of an object when force is applied, serves as an index for the property of an object to deform under force but return to its original shape once the force is removed, and is expressed in the unit of [Pa]. In terms of the physical properties of biomaterials for tissue repair, a higher storage modulus indicates greater elasticity, providing a firmer feel after injection into the body. Particularly for fillers used in aesthetic plastic surgery, a high storage modulus is characterized by providing good volumizing effects and ensuring excellent shape retention.

The term "viscous modulus or loss modulus (G")" as used herein refers to the degree of energy dissipation induced by friction or shear force, and is expressed in the unit of [Pa]. In biomaterials for tissue repair, a higher loss modulus indicates higher viscosity, and particularly for fillers used in aesthetic plastic surgery, this is correlated with the spreadability of the material within the dermal layer after injection.

The term "tan delta (tan δ)" as used herein refers to the ratio of viscosity to elasticity, which is calculated as the loss modulus divided by the storage modulus (G"/G'), representing the degree to which a substance approaches a solid or liquid state. A tan delta greater than 1 indicates a state closer to a liquid, while a value less than 1 indicates a state closer to a solid, and the lower the value, the higher the likelihood that the biomaterial will behave as a single cohesive mass. In biomaterials for tissue repair used in aesthetic plastic surgery, solid-like characteristics imply a reduced likelihood of post-procedural deformation and a lower probability of the implanted filler material migrating within the tissue. For example, a filler with a low tan delta can better withstand the mechanical forces generated by the movement of facial muscles.

The term "complex viscosity" as used herein refers to a function of each measurement frequency and is defined as the geometric mean of the storage modulus and the loss modulus, which represents the resistance to flow and is expressed in the unit of [Pa·S]. A high complex viscosity indicates the firmness of a filler material for tissue repair, and such fillers are suitable for volumizing procedures or for injection into deep layers of skin tissue. Conversely, a lower complex viscosity indicates a softer consistency and a more pronounced fluid-like behavior. Such fillers are intended for injection into the dermal layer or for use in areas where post-procedural deformation is not a significant concern. Furthermore, a high complex viscosity is a critical factor in maintaining a distinct post-procedural contour by reducing the spreadability of the hyaluronic acid hydrogel within the tissue.

Hereinafter, the present disclosure will be described in more detail with reference to the following examples. However, the following examples are provided for illustrative purposes only and are not intended to limit the scope of the present disclosure.

### <Preparation Example 1> Preparation of cryogenically ground particulated human acellular dermal matrix

Skin tissue (harvested from cadavers donated for non-profit patient treatment through a tissue bank) was treated with dispase, which is a neutral protease, at a concentration of 1.0 units/mL. After stirring in an incubator at 37 °C for 60 to 120 minutes, the tissue was washed three times with sterile distilled water to separate the dermal and epidermal layers, followed by the removal of the epidermal layer. The tissue from which the epidermis layer had been removed was treated with a 1% Triton X-100 solution at 30 °C for 100 minutes to decellularize the dermal layer. The tissue was washed three or more times with sterile distilled water to remove processing substances used during the procedures. For lyophilization, the human acellular dermal matrix was frozen at a temperature of -40 °C or lower for at least 2 hours, and then placed in a freeze dryer and dried for 12 hours or longer to remove moisture.

The human acellular dermal matrix was cut into sections of approximately 1 x 1 cm² using a surgical scalpel. Subsequently, 100 g of the human acellular dermal matrix was placed in a micro-grinder and pulverized at a rotation speed of 5,000 rpm for 3 minutes, which is the optimal condition for preventing the denaturation of collagen and elastin. The resulting human acellular dermal matrix was then passed through sieves with 500 µm and 300 µm apertures, respectively, under sterile conditions to thereby prepare particulated human acellular dermal matrix. To prepare smaller particulated human acellular dermal matrix, the particles were pulverized using a cryogenic grinder and then passed through a 100 µm aperture sieve. In summary, through the aforementioned manufacturing process, phADM particles having an average particle size of less than 100 µm, an average particle size in the range of 100 to 300 µm, an average particle size in the range of 300 to 500 µm, and an average particle size exceeding 500 µm were obtained, respectively.

### <Preparation Example 2> On-site preparation of mixed composition of particulated human acellular dermal matrix and hyaluronic acid hydrogel

### (1) Preparation of solution including particulated human acellular dermal matrix

A syringe was filled with 5-10 mL of normal saline, and the particulated human acellular dermal matrix prepared according to Preparation Example 1 was aliquoted into separate syringes according to the desired concentrations. After attaching a Luer-lock connector, plungers on both sides were reciprocated 5 to 10 times to prepare a human acellular dermal matrix solution in which the particulated human acellular dermal matrix and normal saline were mixed.

### (2) Preparation of mixed composition of human acellular dermal matrix solution and hyaluronic acid hydrogel

The human acellular dermal matrix solution and a commercially available hyaluronic acid hydrogel were loaded into separate syringes according to the desired concentration and volume. After attaching a Luer-lock connector, plungers were reciprocated 5 to 10 times to prepare a mixed composition in which the hyaluronic acid hydrogel and the human acellular dermal matrix solution were mixed.

### Injection force evaluation

The injection force was evaluated using ZwickiLine (Zwick/Roell, Germany) equipment. A pre-filled syringe containing the sample to be measured was inserted into a jig, and then a plunger of the syringe was aligned with the center of a stationary plate. Subsequently, the injection force was measured at a rate of 12 mm/min.

### Particle size measurement

The particle size measurement and analysis of the present disclosure were performed using a particle size analysis device (LS13320XR, Beckman Coulter, USA). The sample to be measured was diluted in normal saline at a concentration of 0.1 to 1 wt%, and then measured using a wet mode.

### Measurement of enzymatic degradation of hyaluronic acid

For the degradation test of the present disclosure, an experiment was performed according to the method of JL Reissig et al. (A modified colorimetric method for the estimation of N-acetylamino sugar, J. Biol. Chem. 1955, 217:959-966). An equal mass of the hyaluronic acid hydrogel was dispensed into each test tube, followed by addition of PBS (pH 7.4) containing 10 IU/mL of hyaluronidase (Sigma-Aldrich, USA). The mixture was allowed to react at 37 °C for 24 hours. The enzymatic reaction was terminated by adding 0.1 N HCl (40 vol%). After centrifugation, the supernatant was collected, and the amount of N-acetylglucosamine (NAG) released by degradation was measured using a carbazole assay.

For the carbazole assay, 0.5 mL of the sample was added to a H₂SO₄ reagent in which 0.025 M sodium tetraborate·10H₂O was dissolved, followed by heating at 99 °C for 10 minutes. After cooling the mixture slowly, 0.1 mL of 0.125% carbazole reagent was added, and then the resulting mixture was heated again for 15 minutes, after which the reaction was terminated. For the samples that had undergone the carbazole colorimetric reaction, the absorbance was measured at a wavelength of 530 nm using a UV-vis spectrophotometer. The degree of degradation of the cross-linked product was evaluated by setting the labeled content as 100%, and the degradation rates of the samples were analyzed.

### Characterization of viscoelastic properties

The viscoelastic properties of the present disclosure were characterized using an oscillatory rotational rheometer equipped with parallel plates. The sample to be measured was placed between the plates, and the viscoelasticity was measured while applying a constant horizontal shear stress by rotating one of the plates. A rheometer (MCR302e, Anton Paar Ltd., Austria) was used as the test equipment, and the test items were expressed as storage modulus (G', Pa·s), loss modulus (G", Pa·s), tan delta (G"/G', dimensionless), and complex viscosity (Pa·s). For the measurement, the sample to be measured was loaded after setting the frequency, temperature, shear strain, and measurement gap to 0.1 Hz, 25 °C, 1 %, and 1.0 mm, respectively, and circular parallel plates of 25 mm were used.

### <Experimental Example 1> Evaluation of physical properties of mixed compositions according to average particle sizes of particulated human acellular dermal matrix

Particulated human acellular dermal matrix powder was prepared according to the method of Preparation Example 1, and then filtered through sieves according to the average particle sizes thereof. The average particle size ranges of the filtered particles were categorized into 100 µm or less, 100 to 300 µm, 300 to 500 µm, and 500 µm or more. Each of the four types of powders was mixed with normal saline at a concentration of 10 %, and then according to Preparation Example 2, 1 mL of each resultant solution was mixed with 1 mL of a commercially available hyaluronic acid hydrogel (Sample name: HA1, manufacturer: Hugel, Korea). The content of the particulated human acellular dermal matrix (phADM) in the resulting mixed composition was 5 wt%. The results of comparing the appearances of the respective mixed compositions are shown in FIG. 1 and Table 1.

FIG. 1A illustrates observation images showing the appearances of samples prepared such that the average particle sizes of particulated human acellular dermal matrix in mixed compositions of the particulated human acellular dermal matrix and hyaluronic acid was 100 µm or less, 100 to 300 µm, 300 to 500 µm, and 500 µm or more.

FIG. 1B illustrates optical microscopic observation results showing the shapes of phADM in samples prepared such that the average particle sizes of particulated human acellular dermal matrix in mixed compositions of the particulated human acellular dermal matrix and hyaluronic acid was 100 µm or less, 100 to 300 µm, 300 to 500 µm, and 500 µm or more.

FIG. 1C is a graph comparing the particle size distributions of a hyaluronic acid sample (Sample name: HA1, manufacturer: Hugel, Korea) and four types of human acellular dermal matrix powders with different average particle sizes, within samples prepared such that the average particle sizes of particulated human acellular dermal matrix in mixed compositions of the particulated human acellular dermal matrix and hyaluronic acid was 100 µm or less, 100 to 300 µm, 300 to 500 µm, and 500 µm or more.

The results of FIG. 1C are summarized and shown in Table 1.

**Table 1**

| | HA1 | phADM (<100 µm) | phADM (100-300 µm) | phADM (300-500 µm) | phADM (>500 µm) |
|---|---|---|---|---|---|
| Average particle size (µm, mean ± standard deviation) | 541±6.62 | 45.1±1.25 | 152±0.569 | 663±34.6 | 829±43.6 |
| Mode particle size (µm, mean ± standard deviation) | 534±29.2 | 40.4±2.14 | 140±0.066 | 434±80.7 | 605±32.0 |

As illustrated in FIGS. 1A and 1B, the mixture prepared using particulated human acellular dermal matrix having an average particle size of 100 µm or less exhibited an appearance in which the particulated human acellular dermal matrix was homogeneously distributed within the mixture, whereas the mixture using particles having an average particle size in the range of 100 to 300 µm showed a slightly agglomerated pattern. The solutions prepared using particles having an average size in the range of 300 to 500 µm and particles having an average size exceeding 500 µm exhibited a higher degree of particle agglomeration within the mixtures.

From these results, it can be confirmed that, in case that the average size of the phADM particles is 300 µm or less, the phADM particles are homogeneously mixed with an existing hyaluronic acid hydrogel; however, in case that the average size is 300 µm or more, the particles are distributed relatively broadly, failing to form a homogeneous mixed composition.

As shown in FIG. 1C and Table 1, the mean particle size of HA1 was 541±6.62 µm (mean±standard deviation, n=3) and the mode particle size was 534±29.2 µm (mean±standard deviation, n=3). As a result of measurements according to the sieve aperture size ranges, the mean particle sizes of the four types of powders (100 µm or less, 100 to 300 µm, 300 to 500 µm, and 500 µm or more) were measured to be 45.1±1.25, 152±0.569, 663±34.6, and 829±43.6 µm, respectively (mean ± standard deviation, n=3). In addition, the mode particle sizes thereof were measured to be 40.4±2.14, 140±0.0666, 434±80.7, and 605±32.0 µm, respectively (mean ± standard deviation, n=3). These results indicate that a more homogeneously mixed composition is formed when the average size of the phADM particles is approximately 1/3 of that of hyaluronic acid hydrogel particles.

### <Experimental Example 2> Changes in physical properties of mixed compositions according to concentration of particulated human acellular dermal matrix solutions

Human acellular dermal matrix powder was prepared according to the method of Preparation Example 1, and then the particles were filtered using a sieve with an aperture size of 100 µm, and only particles passing through the sieve were obtained. The powder was mixed with normal saline at concentrations of 2, 10, 20, and 40 wt%, respectively. Subsequently, according to Preparation Example 2, 1 mL of each resulting solution was mixed with 1 mL of a commercially available hyaluronic acid hydrogel (Sample name: HA2, manufacturer: Hugel, Korea) to prepare samples such that the final concentrations of the human acellular dermal matrix powder and the hyaluronic acid (HA2) in the mixed compositions were 1, 5, 10, and 20 wt%, and 1 wt%, respectively.

Each mixture was loaded into a syringe equipped with a commonly used 23G (thin-wall) needle, and the injection forces were measured, and the results thereof are shown in FIG. 2A and Table 2.

FIG. 2A illustrates the results (n=3) of measuring the injection forces for mixed composition samples, including particulated human acellular dermal matrix (1, 5, 10, and 20 wt%) and hyaluronic acid (HA2) (1 wt%), which were loaded into a syringe equipped with a commonly used 23G (thin-wall) needle.

The results of FIG. 2A are summarized and shown in Table 2.

**Table 2**

| | | | | |
|---|---|---|---|---|
| HA2 (wt%) | 1.0 | | | |
| phADM (wt%) | 1.0 | 5.0 | 10.0 | 20.0 |
| Injection force (N, mean ± standard deviation) | 19.5±0.53 | 24.9±0.73 | 30.3±1.26 | 115±0.236 |

As shown in FIG. 2A and Table 2, the mixed composition prepared at a phADM concentration of 1 wt% exhibited the lowest injection force of 19.5 N, while the mixed composition prepared at concentrations of 5 and 10 wt% showed injection force values of 24.9 and 30.3 N, respectively. In contrast, the mixed composition prepared at a concentration of 20 wt% was measured to have an injection force of 115.1 N, exceeding 40 N, confirming that it is difficult to perform syringe injection.

Next, the results of measuring the enzymatic degradation of hyaluronic acid in the mixed compositions of particulated human acellular dermal matrix and hyaluronic acid (HA2) are shown in FIG. 2B and Table 3.

FIG. 2B illustrates the results (n=3) of measuring the degradation rate of hyaluronic acid in mixed composition samples including particulated human acellular dermal matrix (0, 1, 5, and 10 wt%) and hyaluronic acid (HA2) (1 wt%), 24 hours after treatment with hyaluronidase, a hyaluronic acid-degrading enzyme.

The results of FIG. 2B are summarized and shown in Table 3.

**Table 3**

| | | | | |
|---|---|---|---|---|
| HA2 (wt%) | 2.0 | 1.0 | | |
| phADM (wt%) | 0.0 | 1.0 | 5.0 | 10.0 |
| Relative degradation rate of hyaluronic acid (%, mean ± standard deviation) | 93.3±0.841 | 56.5±2.91 | 59.4±5.83 | 64.2±1.68 |

As shown in FIG. 2B and Table 3, 24 hours after hyaluronidase treatment, the existing hyaluronic acid hydrogel (HA2) without phADM exhibited a degradation rate of approximately 93 %, whereas the mixed composition including 1 to 10 wt% of phADM showed a significantly reduced degradation rate ranging from 56 to 64 %, confirming an increase in enzymatic resistance. From these results, it was confirmed that mixing phADM with hyaluronic acid hydrogel increases resistance to hyaluronidase, thereby delaying the degradation rate of a developed mixed composition for tissue repair upon *in vivo* administration, which can significantly improve the duration of efficacy after the procedure.

All the mixed compositions including 1 to 10 wt% of phADM exhibited significantly reduced degradation rates, which is considered preferable. However, given that the degradation rate of hyaluronic acid tended to gradually increase as the content of phADM increased, containing more than 10 wt% of phADM in the mixed composition would be undesirable.

### <Experimental Example 3> Evaluation of physical properties of mixed compositions according to storage modulus (G') of hyaluronic acid hydrogel

The phADM having an average particle size of 100 µm or less prepared according to Preparation Example 1 was mixed with normal saline at a concentration of 10%. To measure the physical properties of the mixed compositions, two types of commercially available hyaluronic acid hydrogels having a storage modulus (G') of 300 Pa or less and exceeding 300 Pa, respectively, before mixing were prepared (G'≤300 Pa, sample name: HA3, manufacturer: Medytox Inc., Korea, n=3; G'>300 Pa, sample name: HA4, manufacturer: Hugel, Korea). According to Preparation Example 2, 1 mL of the phADM solution and 1 mL of each hyaluronic acid hydrogel were mixed, and then the viscoelastic properties of the mixed compositions were analyzed (n=3). The final concentration of phADM was 5.0 wt% and the concentration of hyaluronic acid was 1.0 wt%.

FIG. 3A illustrates the results (n=3) of measuring the storage modulus (G') before and after mixing phADM (100 µm or less) with each of the hyaluronic acids, HA3 and HA4.

FIG. 3B illustrates the results (n=3) of measuring the loss modulus (G") before and after mixing phADM (100 µm or less) with each of the hyaluronic acids, HA3 and HA4.

FIG. 3C illustrates the results of measuring the tan delta before and after mixing phADM (100 µm or less) with each of the hyaluronic acids, HA3 and HA4.

FIG. 3D illustrates the results of measuring the complex viscosity before and after mixing phADM of 100 µm or less with each of the hyaluronic acids, HA3 and HA4 (n=3).

The results of FIGS. 3A to 3D are summarized and shown in Table 4.

**Table 4**

| | HA3 | | HA4 | |
|---|---|---|---|---|
| With or without phADM | X | O | X | O |
| Storage modulus (Pa, mean ± standard deviation) | 289±6.30 | 338±18.8 | 396±12.2 | 371±7.36 |
| Loss modulus (Pa, mean ± standard deviation) | 73.1±2.63 | 66.7±5.60 | 60.3±2.00 | 45.7±2.73 |
| Tan delta (mean ± standard deviation) | 0.25±0.0042 | 0.20±0.0058 | 0.15±0.0095 | 0.12±0.0054 |
| Complex viscosity (Pa·s, mean ± standard deviation) | 469±17.3 | 548±31.1 | 615±13.4 | 595±11.7 |

As shown in FIG. 3 and Table 4,
the storage modulus (G') of the mixture including the hyaluronic acid (HA3) hydrogel having a storage modulus (G') of 300 Pa or less before mixing and the acellular dermal matrix powder solution was found to increase from an average of 289 Pa to 338 Pa, representing a 17.0 % increase after mixing. In contrast, the mixture using the hyaluronic acid (HA4) hydrogel having a storage modulus (G') exceeding 300 Pa before mixing was measured to exhibit a decrease in storage modulus from an average of 396 Pa to 371 Pa, representing a 6.3 % decrease after mixing.

Regardless of the physical properties of the hyaluronic acid hydrogels before mixing, the loss modulus decreased after mixing in all cases: from 73 Pa to 67 Pa (for the hyaluronic acid hydrogel with G'≤300 Pa before mixing), and from 60 Pa to 36 Pa (for the hyaluronic acid hydrogel with G'>300 Pa before mixing).

The tan delta also decreased upon mixing with the phADM solution, from 0.25 to 0.20 (for the hyaluronic acid hydrogel with G'≤300 Pa before mixing) and from 0.15 to 0.12 (for the hyaluronic acid hydrogel with G'>300 Pa before mixing). Therefore, it was confirmed that the mixing of phADM increases the relative elasticity ratio of the existing hyaluronic acid hydrogels.

The complex viscosity also increased from an average of 469 Pa·S to 548 Pa·S for the hyaluronic acid hydrogel having a storage modulus (G') of 300 Pa or less before mixing, while decreasing from an average of 615 Pa·S to 595 Pa·S for the hyaluronic acid hydrogel having a storage modulus exceeding 300 Pa before mixing.

### <Experimental Example 4> Changes in physical properties of mixed compositions using various hyaluronic acid hydrogels having storage modulus (G') of 300 Pa or less

The acellular dermal matrix powder having an average particle size of 100 µm or less prepared according to Preparation Example 1 was mixed with normal saline at a concentration of 10%. To verify the measurement results shown in FIG. 3 and Table 4, three different types of hyaluronic acid hydrogels having a storage modulus (G') of 300 Pa or less before mixing were prepared (n=3), and according to Preparation Example 2, 1 mL of the phADM solution and 1 mL of each hyaluronic acid hydrogel were mixed, followed by analysis (G'≤300 Pa sample name: **HA5**, manufacturer: Medytox Inc., Korea; G'≤300 Pa sample name: **HA6**, manufacturer: Jetema Co., Ltd., Korea; G'≤300 Pa sample name: **HA7**, manufacturer: Medytox Inc., Korea). The final concentration of phADM was 5.0 wt% and the concentration of hyaluronic acid was 1.0 wt%.

FIG. 4A illustrates the results (n=3) of measuring the storage modulus (G') before and after mixing phADM having an average particle size of 100 µm or less with each of the hyaluronic acids, HA5 to HA7.

FIG. 4B illustrates the results (n=3) of measuring the loss modulus (G") before and after mixing phADM having an average particle size of 100 µm or less with each of the hyaluronic acids, HA5 to HA7.

FIG. 4C illustrates the results (n=3) of measuring the tan delta before and after mixing phADM having an average particle size of 100 µm or less with each of the hyaluronic acids, HA5 to HA7.

FIG. 4D illustrates the results (n=3) of measuring the complex viscosity before and after mixing phADM having an average particle size of 100 µm or less with each of the hyaluronic acids, HA5 to HA7.

The results of FIGS. 4A to 4D are summarized and shown in Table 5.

**Table 5**

| | HA5 | | HA6 | | HA7 | |
|---|---|---|---|---|---|---|
| With or without phADM | X | O | X | O | X | O |
| Storage modulus (Pa, mean ± standard deviation) | 138±15.6 | 216±10.5 | 167±2.27 | 191±11.2 | 289±6.30 | 338±18.8 |
| Loss modulus (Pa, mean ± standard deviation) | 40.3±1.52 | 41.3±4.16 | 40.5±0.93 | 26.9±2.86 | 73.1±2.63 | 66.7±5.60 |
| Tan delta (mean ± standard deviation) | 0.29± 0.024 | 0.19± 0.010 | 0.24± 0.0028 | 0.14± 0.0070 | 0.25± 0.0042 | 0.20± 0.0058 |
| Complex viscosity (Pa·s, mean ± standard deviation) | 243±2.65 | 350±17.7 | 271±6.18 | 307±18.2 | 469±17.3 | 548±31.1 |

As shown in FIG. 4 and Table 5,
the storage moduli of HA5, HA6, and HA7 before mixing were measured to be an average of 138, 167, and 289 Pa, respectively. The samples in which the phADM solution was mixed exhibited average values of 216, 191, and 338 Pa, respectively, representing increases of 56.5, 14.4, and 17.0 %.

The loss moduli of HA5, HA6, and HA7 before mixing were measured to be an average of 40, 40, and 73 Pa, respectively; however, upon mixing the phADM solution with each hydrogel, the values were measured to be 41, 27, and 67 Pa, respectively.

The tan delta values of HA5, HA6, and HA7 before mixing were measured to be an average of 0.29, 0.24, and 0.25, respectively; however, upon mixing the phADM solution with each hydrogel, these values were decreased to 0.19, 0.14, and 0.20, respectively.

The complex viscosities of HA5, HA6, and HA7 before mixing were measured to be an average of 243, 271, and 469 Pa·s; however, upon mixing the phADM solution with each hydrogel, these values were increased to 350, 307, and 548 Pa·s, respectively.

From these results, it was confirmed that, when the hyaluronic acid hydrogel having a storage modulus of 300 Pa or less before mixing is mixed with the particulated human acellular dermal matrix having an average particle size of 300 µm or less at a concentration of 10 wt% or less, the resulting mixed composition for tissue repair exhibits increased elasticity and complex viscosity and reduced tan delta.

The foregoing description of the present disclosure is provided for illustrative purposes, and it will be understood by those of ordinary skill in the art to which the present disclosure pertains that the disclosure may be easily modified in many different forms without departing from the technical spirit or essential characteristics of the present disclosure. It is therefore to be understood that the above-described embodiments are illustrative in all aspects and not restrictive.

## Claims

1. A composition for delaying an *in vivo* degradation rate and reinforcing rheological properties of a hyaluronic acid hydrogel used in bio-implantation for tissue repair and having a storage modulus (G') of 300 Pa or less,
the composition comprising particulated human acellular dermal matrix and a medical diluting solvent.

2. The reinforcing composition of claim 1, wherein
the particulated human acellular dermal matrix has an average particle size of 300 µm or less.

3. The reinforcing composition of claim 2, wherein
the average particle size of the particulated human acellular dermal matrix is 100 µm or less.

4. The reinforcing composition of claim 1, wherein
an average particle size of the particulated human acellular dermal matrix is 1/3 or less of an average particle size of hyaluronic acid.

5. The reinforcing composition of claim 1, wherein
the medical diluting solvent is one or more selected from the group consisting of normal saline, distilled water, and phosphate-buffered saline.

6. The reinforcing composition of claim 1, wherein
a mixed composition of the reinforcing composition and the hyaluronic acid hydrogel comprises 10 wt% or less of the particulated human acellular dermal matrix.

7. The reinforcing composition of claim 1, wherein
the rheological properties comprise an increase in storage modulus (G').

8. The reinforcing composition of claim 1, wherein
the *in vivo* degradation rate of the hyaluronic acid hydrogel is reduced by using the reinforcing composition.

9. A bio-implantable composition for tissue repair, comprising particulated human acellular dermal matrix,
the bio-implantable composition being administered in a form of a mixed composition prepared by mixing a solution, in which the particulated human acellular dermal matrix is mixed with a medical diluting solvent, with a hyaluronic acid hydrogel on-site, wherein the hyaluronic acid hydrogel has a storage modulus (G') of 300 Pa or less.

10. The bio-implantable composition of claim 9, wherein
the medical diluting solvent is one or more selected from the group consisting of normal saline, distilled water, and phosphate-buffered saline.

11. The bio-implantable composition of claim 9, wherein
the particulated human acellular dermal matrix has a size of 300 µm or less,
the mixed composition comprises 0.5-10 wt% of the particulated human acellular dermal matrix,
the mixed composition comprises 0.5-2 wt% of the hyaluronic acid hydrogel, and
the size of the particulated human acellular dermal matrix is 1/3 or less of an average particle size of hyaluronic acid.

12. The bio-implantable composition of claim 11, wherein
the size of the particulated human acellular dermal matrix is 200 µm or less,
the mixed composition comprises 1-10 wt% of the particulated human acellular dermal matrix,
the mixed composition comprises 0.7-1.5 wt% of the hyaluronic acid hydrogel, and
the size of the particulated human acellular dermal matrix is 1/3 or less of an average particle size of hyaluronic acid.

13. The bio-implantable composition of claim 12, wherein
the size of the particulated human acellular dermal matrix is 100 µm or less,
the mixed composition comprises 1-5 wt% of the particulated human acellular dermal matrix,
the mixed composition comprises 0.8-1.4 wt% of the hyaluronic acid hydrogel, and
the size of the particulated human acellular dermal matrix is 1/3 or less of an average particle size of hyaluronic acid.

14. The bio-implantable composition of claim 13, wherein
the average particle size of the particulated human acellular dermal matrix is 100 µm or less,
the mixed composition comprises 1-5 wt% of the particulated human acellular dermal matrix,
the mixed composition comprises 0.9-1.3 wt% of the hyaluronic acid hydrogel, and
the size of the particulated human acellular dermal matrix is 1/3 or less of an average particle size of hyaluronic acid.

15. The bio-implantable composition of claim 14, wherein
the size of the particulated human acellular dermal matrix is 100 µm or less,
the mixed composition comprises 1-5 wt% of the particulated human acellular dermal matrix,
the mixed composition comprises 1-1.2 wt% of the hyaluronic acid hydrogel, and
the size of the particulated human acellular dermal matrix is 1/3 or less of an average particle size of hyaluronic acid.

16. A bio-implantable composition for tissue repair, comprising:
a solution in which particulated human acellular dermal matrix and a medical diluting solvent are mixed; and
a hyaluronic acid hydrogel having a storage modulus (G') of 300 Pa or less.
